# EUROPÄISCHE PATENTSCHRIFT

(11) **EP 1 222 207 B1**
(45) Veröffentlichungstag und Bekanntmachung des Hinweises auf die Patenterteilung: **05.12.2007**
(21) Anmeldenummer: 00960664.1
(22) Anmeldetag: 15.09.2000
(51) Int. Cl.: C07K 14/62, C12N 15/10, C12N 15/63, C12N 15/70

(54) **C-PEPTID ZUR VERBESSERTEN HERSTELLUNG VON INSULIN UND INSULINANALOGA**
C-PEPTIDE FOR THE IMPROVED PRODUCTION OF INSULIN AND INSULIN ANALOGUES
PEPTIDE C SERVANT A AMELIORER LA PRODUCTION D'INSULINE ET D'ANALOGUES DE CETTE DERNIERE

(30) Priorität: 02.10.1999 DE 19947456
(43) Veröffentlichungstag der Anmeldung: 17.07.2002
(73) Patentinhaber: Sanofi-Aventis Deutschland GmbH, 65929 Frankfurt am Main (DE)
(72) Erfinder: HABERMANN, Paul, 65817 Eppstein (DE); ERTL, Johann, 65520 Bad Camberg (DE); MEIWES, Johannes, 65510 Idstein (DE); SEIPKE, Gerhard, 65719 Hofheim (DE)
(86) Internationale Anmeldenummer: PCT/EP2000/009017
(87) Internationale Veröffentlichungsnummer: WO 2001/025278

(56) Entgegenhaltungen:
- EP-A- 0 704 527
- EP-A- 0 821 006
- EP-A- 0 885 961
- US-A- 4 430 266
- SCHMIDT M ET AL: "Temperature-induced production of recombinant human insulin in high-cell density cultures of recombinant Escherichia coli" JOURNAL OF BIOTECHNOLOGY,NL,ELSEVIER SCIENCE PUBLISHERS, AMSTERDAM, Bd. 68, Nr. 1, 5. Februar 1999 (1999-02-05), Seiten 71-83, XP004157320 ISSN: 0168-1656
- VOLLENWEIDER ET AL: "Processing of proinsulin by furin" DIABETES,NEW YORK, NY,US, Bd. 44, September 1995 (1995-09), Seiten 1075-1080, XP002125739 ISSN: 0012-1797
- YANAGITA MASAHIKO ET AL: "Processing of mutated proinsulin with tetrabasic cleavage sites to mature insulin reflects the expression of furin in nonendocrine cell lines." ENDOCRINOLOGY, Bd. 133, Nr. 2, 1993, Seiten 639-644, XP000987087 ISSN: 0013-7227
- STEINER D F ET AL: "The role of prohormone convertases in insulin biosynthesis: Evidence for inherited defects in their action in man and experimental animals." DIABETE & METABOLISME, Bd. 22, Nr. 2, 1996, Seiten 94-104, XP000987091 ISSN: 0338-1684

### SEQUENZPROTOKOLL

<110> Aventis Pharma Deutschland GmbH
<120> C-Peptid zur verbesserten Herstellung von Insulin und Insulinanaloga
<130> 1999/L059
<140> 19947456.7
   <141> 1999-10-02
<160> 13
<170> PatentIn Ver. 2.1
<210> 1
   <211> 87
   <212> DNA
   <213> Künstliche Sequenz
<220>
   <223> Beschreibung der künstlichen Sequenz:DNA kodierend für C-Peptid Variante
<400> 1
   cgcgatgttc ctcaggtgga gctgggcggg ggccctggcg caggcagcct gcagcccttg 60
   gcgctggagg ggtccctgca gaagcgc                                     87
<210> 2
   <211> 29
   <212> PRT
   <213> Künstliche Sequenz
<220>
   <223> Beschreibung der künstlichen Sequenz:Variante C-Pep-Insulin
<400> 2
   Arg Asp Val Pro Gln Val Glu Leu Gly Gly Gly Pro Gly Ala Gly Ser
    1               5                  10                  15
   Leu Gln Pro Leu Ala Leu Glu Gly Ser Leu Gln Lys Arg
               20                  25
<210> 3
   <211> 45
   <212> DNA
   <213> Künstliche Sequenz
<220>
   <223> Beschreibung der künstlichen Sequenz:Primer PINT 358fIII
<400> 3
   cccaagaccc gcgatgttcc tcaggtggag ctgggcgggg gccct 45
<210> 4
   <211> 4
   <212> PRT
   <213> Künstliche Sequenz
<220>
   <223> Beschreibung der künstlichen Sequenz:Proteinteilsequenz aus PINT 358fIII
<400> 4
   Arg Asp Val Pro
    1
<210> 5
   <211> 40
   <212> DNA
   <213> Künstliche Sequenz
<220>
   <223> Beschreibung der künstlichen Sequenz:Primer PINT 358revII
<400> 5
   cagctccacc tgaggaacat cgcgggtctt gggtgtgtag 40
<210> 6
   <211> 31
   <212> DNA
   <213> Künstliche Sequenz
<220>
   <223> Beschreibung der künstlichen Sequenz:Primer Salforward
<400> 6
   tacacacccg agacccgcga tgttcctcag g 31
<210> 7
   <211> 32
   <212> DNA
   <213> Künstliche Sequenz
<220>
   <223> Beschreibung der künstlichen Sequenz:Primer 329rev
<400> 7
   cctgaggaac atcgcgggtc tcgggtgtgt ag 32
<210> 8
   <211> 91
   <212> PRT
   <213> Künstliche Sequenz
<220>
   <223> Beschreibung der künstlichen Sequenz:Variante Insulinvorläufer
<400> 8
   Met Ala Thr Thr Ser Thr Gly Asn Ser Ala Arg Phe Val Lys Gln His
    1               5                  10                  15
   Leu Cys Gly Ser His Leu Val Glu Ala Leu Tyr Leu Val Cys Gly Glu
               20                  25                  30
   Arg Gly Phe Phe Tyr Thr Pro Glu Thr Arg Asp Val Pro Gln Val Glu
           35                  40                  45
   Leu Gly Gly Gly Pro Gly Ala Gly Ser Leu Gln Pro Leu Ala Leu Glu
       50                  55                  60
   Gly Ser Leu Gln Lys Arg Gly Ile Val Glu Gln Cys Cys Thr Ser Ile
   65                  70                  75                  80
   Cys Ser Leu Tyr Gln Leu Glu Asn Tyr Cys Asn
                   85                  90
<210> 9
   <211> 24
   <212> DNA
   <213> Künstliche Sequenz
<220>
   <223> Beschreibung der künstlichen Sequenz:Primer 140drev
<400> 9
   aaaggtcgac tattagccgc agta 24
<210> 10
   <211> 13
   <212> PRT
   <213> Künstliche Sequenz
<220>
   <223> Beschreibung der künstlichen Sequenz:Proteinteilsequenz aus PINT 349a
<400> 10
   Val Gln Pro Val Asp Arg His His Thr Lys Pro Thr Tyr
    1               5                   10
<210> 11
   <211> 40
   <212> DNA
   <213> Künstliche Sequenz
<220>
   <223> Beschreibung der künstlichen Sequenz:Primer 349a
<400> 11
   cacctgagga acatcgcggt ggtgggtctt gggtgtgtag 40
<210> 12
   <211> 13
   <212> PRT
   <213> Künstliche Sequenz
<220>
   <223> Beschreibung der künstlichen Sequenz:Proteinteilsequenz aus PINT 349b
<400> 12
   Pro Lys Thr His His Arg Asp Val Pro Gln Val Glu Leu
    1               5                   10
<210> 13
   <211> 40
   <212> DNA
   <213> Künstliche Sequenz
<220>
   <223> Beschreibung der künstlichen Sequenz:Pirmer PINT 349b
<400> 13
   acccaagacc caccaccgcg atgttcctca ggtggagctg 40

## Beschreibung

Die vorliegende Erfindung betrifft ein synthetisches Derivat des C - Peptides aus Proinsulin. Proinsulin, enthaltend dieses Derivat, hat auf verschiedene Art und Weise bessere Eigenschaften als gewöhnliches Affenproinsulin, insbesondere verbessert sich die Endausbeute des jeweiligen Insulinderivats bei dessen gentechnischer Herstellung.

Die Zahl der Erkrankungen an Diabetes nimmt weltweit stetig zu. Proportional dazu steigt der Bedarf an Insulin oder Derivaten des Insulin. Damit besteht die Aufgabe, die bestehenden Verfahren hinsichtlich der Ausbeute an Wirkstoff zu optimieren. Im Europäischen Patent EP-B1 0 489 780 ist ein Verfahren zur Herstellung von Insulin oder Derivaten hiervon vorgeschlagen. Die dort beschriebenen Vektoren dienen zur Herstellung von Humaninsulin mit dem Plasmid pINT90d oder als Ausgangsplasmide zur Konstruktion des in der Europäischen Patentanmeldung EP-A 0 821 006 beschriebenen Plasmides pINT302d, das zur Herstellung eines His(B31) His(B32) Gly(A21) - Insulinderivates dient, oder zur Konstruktion des in der Europäischen Patentanmeldung EP-A 0 885 961 beschriebenen Vektors pINT329d, der zur Herstellung des Lys(B3) Glu(B29) Insulinderivates dient.

Es wurde nun gefunden, daß besonders vorteilhafte Proinsulinderivate solche der Formel I sind,

Fus-B(1-30)-RDVP-Yₙ-A(1-21) (I);

wobei
- Fus: ein optional vorhandener Fusionsanteil beliebiger Sequenz ist;
- B(1-30): die B-Kette von humanem Insulin ist,
- Y: für eine Aminosäurekette steht, welche mit einer basischen Aminosäure C-terminal endet;
- n: gleich 2 bis 50 ist und die Länge der Aminosäurekette Y angibt; und
- A(1-21): die A-Kette von humanem Insulin ist,
und die A- und/oder die B-Kette durch Aminosäureaustausche, Deletionen und/oder Additionen modifiziert sein können. Überraschend werden dabei je nach Zusammensetzung der Insulin A - bzw. - B - Kette gleiche und voneinander verschiedene Vorteile beobachtet.

Verbindet man die humane B- mit der humanen A-Kette über das vorteilhafte C-Peptid, so entsteht ein Proinsulin, das sich bzgl. Expressionsausbeute wie Wildtyp- Proinsulin verhält, dessen enzymatische Prozessierung zu Insulin aber leichter steuerbar ist, so daß keine störende Spuren von um Arginin verlängerter B - Kette entstehen, die bei der Herstellung zum Arzneimittel abgetrennt werden müssen, wodurch Ausbeuteverluste auftreten.

Verbindet man eine um di - Histidin C- terminal verlängerte B- Kette mit der A- Kette von Humaninsulin, die in Position A21 Glycin enthält, mit dem erfindungsgemäßen C-Peptid, so beobachtet man eine um ca. 20% höhere Expressionssausbeute im Vergleich zu den mit dem Plasmid pINT90d erzielbaren Ausbeuten und eine fast fünffach höhere Ausbeute, als dies mit dem Plasmid pINT302d beobachtet wurde. Zudem ist die Steuerung der enzymatischen Prozessierung ebenso vereinfacht, wie vorher beschrieben wurde.

Verbindet man eine Lys(B3) Glu (B29) modifizierte B- Kette mit der A - Kette von Humaninsulin über das modifizierte C- Peptid, so beobachtet man verbesserte Faltungseigenschaften des Proinsulinderivates im Vergleich zu dem von plNT329d kodierten Proinsulin. Die Ausbeute an Rohfusionsprotein ist erhöht und erreicht ein gleiches Niveau wie mit dem Plasmid pINT90d beobachtet wird. Zudem ist die Steuerung der enzymatischen Prozessierung vereinfacht.

Eine besonders vorteilhafte Ausführungsform des neuen C -Peptids ist durch folgende Aminosäuresequenz charakterisiert : A L E G S L Q K R (SEQ ID NO.: 2)

Ebenfalls angegeben ist eine von vielen möglichen DNA-Sequenzen, die für das angegebene C-Peptid kodiert.

Ein Gegenstand der Erfindung ist ein Vorläufer von humanem Insulin oder eines Insulinanalogons der Formel I

Fus-B(1-30)-RDVP-Yₙ-A(1-21) (I);

wobei
- Fus: ein optional vorhandener Fusionsanteil beliebiger Sequenz ist;
- B(1-30): die B-Kette von humanem Insulin ist,
- Y: für eine Aminosäurekette steht, welche mit einer basischen Aminosäure C-terminal endet;
- n: gleich 2 bis 50 ist und die Länge der Aminosäurekette Y angibt; und
- A(1-21): die A-Kette von humanem Insulin ist,
und die A- und/oder die B-Kette durch Aminosäureaustausche, Deletionen und/oder Additionen modifiziert sein können, insbesondere wobei Yₙ für die Aminosäuren 5 bis 35 des C-Peptids von humanem oder Affen-Insulin steht, bevorzugt, wobei Yₙ für die Aminosäuren 11 bis 35 von humanem Insulin steht.

Ein weiterer Gegenstand der Erfindung sind Vorläufer wie oben beschrieben, wobei die B-Kette von humanem Insulin die Modifikationen Lys(B3) Glu(B29) oder wobei die B- und A-Ketten von humanem Insulin die Modifikationen His(B31) His(B32) Gly(A21) enthalten.

Darüberhinaus ist Gegenstand der Erfindung eine DNA, kodierend für einen Vorläufer wie oben beschrieben.

Ebenfalls Gegenstand der Erfindung ist ein Vektor, enthaltend eine DNA, kodierend für einen Vorläufer wie oben beschrieben, vorzugsweise, dadurch gekennzeichnet, daß der Vektor ein Expressionsvektor geeignet zur Expression in E.coli ist.

Ein weiterer Gegenstand der Erfindung ist eine E.coli-Zelle, enthaltend einen Vektor wie oben beschrieben.

Ein weiterer Gegenstand der Erfindung ist ein Verfahren zur Herstellung eines Vorläufers wie oben beschrieben, wobei
(a) eine DNA wie oben beschrieben in einen Vektor wie oben beschrieben eingebracht wird;
(b) der Vektor aus (a) in eine *E.coli*-Zelle eingeschleust wird;
(c) die *E.coli*-Zelle aus (b) enthaltend den Vektor aus (a) zur Expression benutzt wird; und
(d) der Vorläufer aus dem Kulturüberstand isoliert wird.

Darüberhinaus ist Gegenstand der Erfindung ein Verfahren zur Herstellung einer DNA wie oben beschrieben, wobei
(a) ausgehend von der cDNA des humanen oder Affen-Insulin mittels PCR und anderer molekularbiologischen Techniken diese DNA erzeugt und
(b) isoliert wird.

Ein weiterer Gegenstand der Erfindung ist ein Verfahren zur Herstellung von humanem Insulin, oder eines Insulinanalogons, wobei
(a) ein Vorläufer wie oben beschrieben gemäß dem Verfahren wie oben beschrieben erzeugt wird;
(b) der Vorläufer gemäß (a) unter geeigneten Bedingungen so gefaltet wird, daß sich die Disulfidbrücken wie in Humaninsulin ausbilden können, und der RDVP-Yₙ-Teil und gegebenenfalls der Fusionsanteil Fus enzymatisch entfernt werden; und
(c) das humane Insulin oder das Insulinanalogon aufgereinigt wird.

Ein weiterer Gegenstand der Erfindung ist die Verwendung eines Vorläufers wie oben beschrieben zur Herstellung von Insulin oder eines Insulinanalogons, vorzugsweise wobei die Herstellung von Insulin oder einem Insulinanalogon gemäß dem Verfahren wie oben beschrieben erfolgt.

Des weiteren ist Gegenstand der Erfindung die Verwendung einer DNA wie oben beschrieben zur Herstellung eines Vorläufers wie oben beschrieben.

Auch ist Gegenstand der Erfindung die Verwendung eines Vektors wie oben beschrieben zur Herstellung eines Vorläufers wie oben beschrieben.

Ein weiterer Gegenstand der Erfindung ist die Verwendung einer E.coli-Zelle wie oben beschrieben zur Herstellung eines Vorläufers wie oben beschrieben.

Die Erfindung wird nun anhand der Beispiele näher erläutert, ohne sich aber darauf zu beschränken.

### Beispiel 1: Expression von Proinsulinderivaten

Die Expression erfolgt wie in EP-B1 0 489 780 beschrieben. Dabei können bei Fermentation im großen Volumen Modifikationen eingeführt werden. Beim Vergleich der Expressionsraten werden aber stets die gleichen Bedingungen eingehalten.

Für das Arbeiten in größeren Maßstäben gilt folgendes allgemeines Fermentationsrezept, das beispielhaft für ein Volumen von 7,5 Litern beschrieben wird:

| | |
|---|---|
| Fermentationsvolumen : | 7,5 l |
| Sterilisationsbedingungen : | 121°C, 20 Minuten, bei pH 3,5, nach Sterilisation mit NH₃ auf 7,0 eingestellt. |
| Fermentationstemperatur : | 37°C |
| pH-Regulierung : | pH 7,0, Einstellung mit 25% Ammoniakwasser |
| Rührerdrehzahl | : 1500 UpM |
| Belüftung | : 15 Nl/min (2 vvm) |
| Dauer | : ca.24h |
| Feeding | : 65%ige Glucose wurde ab einem OTR-Wert von 200 mmoll⁻¹h⁻¹ mit einer konstanten Rate von 12 gl⁻¹h⁻¹ zudosiert. |
| Vorkultur | : Eine Schüttelkultur wurde mit einer Seed-Ampulle beimpft und für 3 - 4h bei 37°C , 250 UpM bis zu einer OD A₅₄₀ ~ 1 inkubiert. |
| Beimpfung | : Die Fermenter wurden mit etwa 40 ml Vorkultur angeimpft. |
| Induktion | : Bei einer A₅₄₀ von ≥40 mit 40 mg/l (300 mg / Fermenter) Indolpropionsäure gelöst in ca. 10 ml einer wäßrigen Na₂CO₃-Lsg. (mit 0,17g Na₂CO₃). |

Hierbei bedeuten NI = Normliter, vvm = Volumen/Volumen/Minute und OTR = Oxygen Transfer Rate.

### Fermentationsmedien:

| Rezeptnummer GAI 100/95-000: | Menge g/l |
|---|---|
| Glucose-1-hydrat, D(+) min. 80% | 44 |
| Citronensäure-1-hydrat | 3,48 |
| Ammoniumsulfat min.95% | 6,0 |
| Phosphorsäure, ortho, 85% | 2,99 |
| Di-Kalium-hydrogenphosphat | 1,18 |
| Natriumsulfat | 3,0 |
| Magnesiumsulfat-7-hydrat, minimum 98% | 2,0 |
| Eisen(III)-sulfat x H₂O | 0,5 |
| Spurenelementlsg.: RL 1/85-000 | 1,0 ml |
| Thiamin-HCl | 0,005 |
| Desmophen 3600 | 0,5 |

### Spurenelementlsg.:

| RL 1/85-000 | Menge g/l |
|---|---|
| Kupfer(II)sulfat-5-hydrat | 1,6 |
| Kaliumjodid | 4,0 |
| Ammoniummolybdat-4-hydrat | 8,0 |
| Mangan(II)-sulfat-1-hydrat | 12,3 |
| Zinksulfat-7-hydrat | 16 |
| Borsäure | 20 |

### Medium im Schüttelkolben:

| | Menge g/l |
|---|---|
| Hefeextrakt | 8,0 |
| Glucose | 1,0 |
| NaCl | 3,5 |
| KH₂PO₄ | 1,32 |
| K₂HPO₄ | 3,68 |

### Beispiel 2: Herstellung von Insulinen

Die Herstellung von Insulinen erfolgt gemäß bekannter Methoden, wie sie z.B. in EP-B1 0 489 780 oder EP-A 0 885 961 beschrieben oder diskutiert wurden. Bevorzugt zur Faltung und Aufarbeitung des jeweiligen Fusionsproteins ist dabei die in EP-B1 0 668 282 (s. Beispiel 2) beschriebene Methode. Dabei kann nach Faltung entsprechend EP 0 288 809 der Ansatz vor weiterer Aufarbeitung filtriert werden.

### Beispiel 3: Konstruktion des Plasmids plNT358d kodierend für das in bezug auf die C-Kette derivatisierte humane Proinsulin B - RDVP C₁₁₋₃₅- A

Zur Herstellung des Plasmides wurden die in EP-B1 0 489 780 beschriebenen Primer Tir und Insu11 verwendet. Zusätzlich wurden zwei neue Primersequenzen synthetisiert.

Primer PINT358fIII hat folgende Sequenz:
5'- CCC AAG ACC CGC **GAT GTT CCT** CAG GTG GAG CTG GGC GGG GGC CCT - 3' (SEQ ID NO.:3)
B28 B29 B30 Arg Asp Val Pro C11 C12 C13 C14 C15 C16 C17 C18 (SEQ ID NO.: 4)
Primer PINT358revll hat die Sequenz:
5'- CAGCTCCACCTGAGGAACATCGCGGGTCTTGGGTGTGTAG - 3' (SEQ ID NO.: 5)

Mit DNA des Plasmides plNT90d als Matrize werden mit den Primerpaaren Tir/PINT358revll und Insu11/PINT358fIII entsprechend EP-B1 0 489 780 je eine PCR durchgeführt. Aliquots der Produkte der beiden Reaktionen werden kombiniert und zusammen mit dem Primerpaar Tir/Insu11 in einer dritten PCR eingesetzt. Das Produkt dieser Reaktion wird mit den Enzymen Sall/Ncol doppelverdaut und das Produkt dieses Restriktionsverdaus nach Reinigung in die mit Ncol/Sall geöffnete Vektor DNA des ebenfalls in EP-B1 0 489 780 beschriebenen Plasmides pINT91d insertiert.Das so konstruierte Plasmid erhält die Bezeichnung pINT358d. Die Struktur wird mittels DNA - Sequenzanalyse bestätigt. Kompetente E.coli Zellen werden mit DNA des Plasmides transformiert. Die Expression des Proinsulins in Bakterien erfolgt gemäß Beispiel 1. Nach Faltung gemäß Beispiel 2 erfolgt die enzymatische Umsetzung des Proinsulin zu Insulin und weitere Reinigung gemäß EP-B1 0 347 781. Dabei kann im Vergleich zu dem aus plNT90d abgeleiteten Verfahren im lonentauscherchromatographieschritt eine Randfraktion zusätzlich aufgefangen werden, da diese nicht mit Arg(B31) - Insulin kontaminiert ist.

### Beispiel 4: Konstruktion des Plasmides plNT362d zur Herstellung von Lys(B3)Glu(B29) - RDVP -C₁₁₋₃₅-Proinsulin

Zur Konstruktion des Plasmides werden DNA der Plasmide plNT329d und plNT358d als Matrize und die Primer Tir und Insu11 benötigt. Zusätzlich werden zwei neue Primer Salforward und 329rev synthetisiert.
Primer Salforward hat die Sequenz :
5'- TACACACCCGAGACC**CGCGATGTTCCTCAGG**-3' (SEQ ID NO.: 6)

Dabei markiert der fett gedruckte Sequenzabschnitt die Sequenz , die mit dem Plasmid plNT358d hybridisiert während der restliche Teil homolog zu Sequenzen des die B-Kette kodierenden Abschnittes des Plasmides plNT329d ist.

Primer 329rev hat folgende Sequenz :
5' - CCTGAGGAACATC**GCGGGTCTCGGGTGTGTAG** - 3' (SEQ ID NO.: 7)

Dabei markiert der fett gedruckte Abschnitt die Region, die homolog zu dem Antisense Strang ist, der das Ende der B-Kette bildet und das Triplett für Arginin im Plasmid plNT329d beschreibt. Die restliche Sequenz hybridisiert mit Plasmid pINT358d. Zwei PCR - Ansätze werden durchgeführt Dabei dient die DNA des Plasmides plNT329d als Matrize für das Primerpaar Tir / 329rev und pINT358d DNA als Matrize für das Paar Salforward / Insu11.

Beide Reaktionen resultieren in Fragmenten, die sich um die Sequenz des Primer Salforward überlappen. Damit können die beiden Fragmente in einer dritten PCR vereinigt werden und mit Hilfe der Primer Tir und Insu11 zu einer DNA - Sequenz zusammengefügt werden , die das Insulinanalogon kodiert. Dieses Reaktionsprodukt wird mit den Restriktionsenzymen Ncol/Sall gespalten und anschließend in das Sall/Ncol geöffnete Vektorfragment aus pINT91d insertiert. Kompetente Zellen des Stammes *E.coli* K12 MM294 werden mit dem entsprechenden Ligationsansatz transformiert. Plasmid DNA wird von Transformanten isoliert und charakterisiert. Das richtige Plasmid erhält die Bezeichnung pINT362d.

Nach Expression wird eine zu pINT90d vergleichbare Rohausbeute an Fusionsprotein beobachtet. Jedoch ist die beobachtete Faltungsausbeute im Vergleich zu pINT329d um ca 40% besser.

Die Struktur des Fusionsproteins kodiert durch pINT362d sieht wie folgt aus:

### Beispiel 5: Konstruktion des Plasmides pINT349d zur Herstellung von His(B31) His(B32) Gly(A21)- RDVP -C₁₁₋₃₅-Proinsulin

Zunächst wird das Plasmid pINT140d, dessen DNA das Insulinanalogon Gly (A21)-Insulin kodiert, hergestellt.

Dazu werden zwei Oligonukleotide zur Verwendung in einer PCR als Primer benötigt :

Oligonucleotid Tir wird als 'Sense' und Oligonukleotid 140drev als 'Antisense'-Primer verwendet:

Beide Primer werden in einer Standard PCR mit DNA des Plasmides pINT90d eingesetzt. Das Reaktionsprodukt wird entsprechend EP-B1 0 489 780 mit den Restriktionsenzymen Ncol und Sall umgesetzt und anschließend in den entsprechend geöffneten Vektor pINT69d eingesetzt. Es entsteht das Plasmid pINT140d das nach Transformation in *E.coli* K12 MM294 reisoliert und mittels Restriktions - und Sequenzanalyse charakterisiert wird.

Ausgehend von der DNA des Plasmides pINT140d werden zwei PCR - Ansätze durchgeführt. Die erste Reaktion benutzt den Primer Tir und als reversen Primer PINT349a mit folgender Sequenz:

Dabei bezeichnen die mit Stern markierten Sequenzen die Codone für die neu eingeführten Aminosäuren.

Die zweite PCR wird mit den Primern Inu11 und PINT349b durchgeführt. Primer PINT349b hat die Sequenz :

Von Position 34 bis Position 1 der DNA - Sequenz ist der Primer komplementär zu PINT349a. Daher können die Reaktionsprodukte der beiden PCR in einer dritten PCR mit den Primer Tir und Insu11 zu dem DNA - Fragment zusammengefügt werden, das für das gewünschte Proinsulinderivat kodiert. Das Produkt dieser Reaktion wird wie beschrieben mit den Enzymen Ncol und Sall umgesetzt und in das mit diesen Enzymen geöffnete Vektorfragment pINT91d eingesetzt und nach *E.coli* K12 transformiert. Nach Charakterisierung der Plasmide aus Transformanten erhalten richtige Plasmidkonstruktionen die Bezeichnung pINT349d.

Exprimiert man das Fusionprotein, so zeigt sich ein deutliche Erhöhung der Ausbeute an Fusionsprotein. Die Ausbeute ist überraschend ca 20% höher als dies mit pINT90d zu erzielen ist und ca. 5 mal größer als mit Plasmid pINT30d erzielt. Die Faltungsrate ist dabei vergleichbar mit der bei Affenpräproinsulin, kodiert von pINT90d, erzielten Rate.

## Patentansprüche

1. Vorläufer von humanem Insulin oder eines Insulinanalogons der Formel I
Fus-B(1-30)-RDVP-Yₙ-A(1-21) (I);
wobei
Fus ein optional vorhandener Fusionsanteil beliebiger Sequenz ist;
B(1-30) die B-Kette von humanem Insulin ist,
Y für eine Aminosäurekette steht, welche mit einer basischen Aminosäure C-terminal endet;
n gleich 2 bis 50 ist und die Länge der Aminosäurekette Y angibt; und
A(1-21) die A-Kette von humanem Insulin ist,
und die A- und/oder die B-Kette durch Aminosäureaustausche, Deletionen und/oder Additionen modifiziert sein können.

2. Vorläufer gemäß Anspruch 1, wobei Yₙ für die Aminosäuren 5 bis 35 des C-Peptids von humanem oder Affen-Insulin steht.

3. Vorläufer gemäß Anspruch 1, wobei Yₙ für die Aminosäuren 11 bis 35 von humanem Insulin steht.

4. Vorläufer gemäß einem der Ansprüche 1 bis 3, wobei die B-Kette von humanem Insulin die Modifikationen Lys(B3)Glu(B29) enthält.

5. Vorläufer gemäß einem der Ansprüche 1 bis 3, wobei die B- und A-Ketten von humanem Insulin die Modifikation His(B31)His(B32)Gly(A21) enthalten.

6. DNA, kodierend für einen Vorläufer gemäß einem der Ansprüche 1 bis 5.

7. Vektor, enthaltend eine DNA gemäß Anspruch 6.

8. Vektor gemäß Anspruch 7, **dadurch gekennzeichnet, daß** der Vektor ein Expressionsvektor geeignet zur Expression in *E.coli* ist.

9. *E.coli*-Zelle, enthaltend einen Vektor gemäß Anspruch 8.

10. Verfahren zur Herstellung eines Vorläufers gemäß einem der Ansprüche 1 bis 5, wobei
a) eine DNA gemäß Anspruch 6 in einen Vektor gemäß Anspruch 7 eingebracht wird;
b) der Vektor aus (a) in eine E.coli-Zelle eingeschleust wird;
c) die *E.coli*-Zelle aus (b) enthaltend den Vektor aus (a) zur Expression benutzt wird; und
d) der Vorläufer aus dem Kulturüberstand isoliert wird.

11. Verfahren zur Herstellung einer DNA gemäß Anspruch 6, wobei
a) ausgehend von der cDNA des humanen oder Affen-Insulin mittels PCR und anderer molekularbiologischen Techniken die DNA gemäß Anspruch 6 erzeugt und
b) isoliert wird.

12. Verfahren zur Herstellung von humanem Insulin, oder eines Insulinanalogons, wobei
a) ein Vorläufer gemäß dem Verfahren nach Anspruch 10 erzeugt wird;
b) der Vorläufer gemäß (a) unter geeigneten Bedingungen so gefaltet wird, daß sich die Disulfidbrücken wie in Humaninsulin ausbilden können, und der RDVP-Yₙ-Teil und gegebenenfalls der Fusionsanteil Fus enzymatisch entfernt werden; und
c) das humane Insulin oder das Insulinanalogon aufgereinigt wird.

13. Verwendung eines Vorläufers gemäß einem der Ansprüche 1 bis 5 zur Herstellung von Insulin oder eines Insulinanalogons.

14. Verwendung gemäß Anspruch 13, wobei die Herstellung von Insulin oder einem Insulinanalogon gemäß dem Verfahren des Anspruchs 12 erfolgt.

15. Verwendung einer DNA gemäß Anspruch 6, zur Herstellung eines Vorläufers gemäß einem der Ansprüche 1 bis 5.

16. Verwendung eines Vektors gemäß Anspruch 8 zur Herstellung eines Vorläufers gemäß einem der Ansprüche 1 bis 5.

17. Verwendung einer E.coli-Zelle gemäß Anspruch 9 zur Herstellung eines Vorläufers gemäß einem der Ansprüche 1 bis 5.

18. Verwendung gemäß einem der Ansprüche 15,16 oder 17, wobei die Herstellung des Vorläufers gemäß dem Verfahren des Anspruchs 10 erfolgt.

## Claims

1. A precursor of human insulin or of an insulin analog of the formula I
Fus-B(1-30)-RDVP-Yₙ-A(1-21) (I);
where
Fus is an optionally present fusion portion of any suitable sequence;
B(1-30) is the B chain of human insulin,
Y is an amino acid chain which terminates with a basic amino acid at the C terminus;
n is from 2 to 50 and indicates the length of the amino acid chain Y; and
A(1-21) is the A chain of human insulin,
and the A chain and/or the B chain can be modified by amino acid exchanges, deletions and/or additions.

2. A precursor as claimed in claim 1, where Yₙ is amino acids 5 to 35 of the C peptide of human or monkey insulin.

3. A precursor as claimed in claim 1, where Yₙ is amino acids 11 to 35 of human insulin.

4. A precursor as claimed in any of claims 1 to 3, where the B chain of human insulin comprises the modifications Lys(B3)Glu(B29).

5. A precursor as claimed in any of claims 1 to 3, where the B and A chains of human insulin comprise the modification His(B31)His(B32)Gly(A21).

6. A DNA coding for a precursor as claimed in any of claims 1 to 5.

7. A vector comprising a DNA as claimed in claim 6.

8. A vector as claimed in claim 7, wherein the vector is an expression vector suitable for expression in *E.coli.*

9. An *E.coli* cell comprising a vector as claimed in claim 8.

10. A process for preparing a precursor as claimed in any of claims 1 to 5, where
a) a DNA as claimed in claim 6 is introduced into a vector as claimed in claim 7;
b) the vector from (a) is introduced into an E.coli cell;
c) the *E.coli* cell from (b) comprising the vector from (a) is used for expression; and
d) the precursor is isolated from the culture supernatant.

11. A process for preparing a DNA as claimed in claim 6, where
a) the DNA as claimed in claim 6 is produced starting from the cDNA of human or monkey insulin by means of PCR and other molecular biology techniques, and
b) is isolated.

12. A process for preparing human insulin or an insulin analog, where
a) a precursor is produced by the process as claimed in claim 10;
b) the precursor from (a) is folded under suitable conditions so that the disulfide bridges can form as in human insulin, and the RDVP-Yₙ part and, where appropriate, the fusion portion Fus are deleted enzymatically; and
c) the human insulin or the insulin analog is purified.

13. The use of a precursor as claimed in any of claims 1 to 5 for preparing insulin or an insulin analog.

14. The use as claimed in claim 13, where insulin or an insulin analog is prepared by the process as claimed in claim 12.

15. The use of a DNA as claimed in claim 6 for preparing a precursor as claimed in any of claims 1 to 5.

16. The use of a vector as claimed in claim 8 for preparing a precursor as claimed in any of claims 1 to 5.

17. The use of an *E.coli* cell as claimed in claim 9 for preparing a precursor as claimed in any of claims 1 to 5.

18. The use as claimed in any of claims 15, 16 or 17, wherein the precursor is prepared by the process of claim 10.

## Revendications

1. Précurseur de l'insuline humaine ou d'un analogue de l'insuline de formule I :
Fus-B(1-30) -RDVP-Yₙ-A(1-21) (I);
dans laquelle :
Fus représente une partie de fusion éventuellement présente d'une séquence quelconque;
B(1-30) représente la chaîne B de l'insuline humaine;
T représente une chaîne d'acides aminés, qui se termine par un acide aminé basique au niveau de l'extrémité C-terminale;
n vaut 2 à 50 et représente la langueur de la chaîne d'acides aminés T; et
A(1-21) représente la chaîne A de l'insuline humaine;
les chaînes A et/ou B pouvant être modifiées par des échanges d'acides aminés, des délétions et/ou des additions.

2. Précurseur selon la revendication 1, dans lequel Yₙ représente les acides aminés 5 à 35 du peptide C de l'insuline d'origine humaine ou simienne.

3. Précurseur selon la revendication 1, dans lequel Yₙ représente les acides aminés 11 à 35 de l'insuline humaine.

4. Précurseur selon l'une quelconque des revendications 1 à 3, dans lequel la chaîne B de l'insuline humaine contient les modifications Lys(B3)Glu(B29).

5. Précurseur selon l'une quelconque des revendications 1 à 3, dans lequel les chaînes B et A de l'insuline humaine contiennent les modifications His(B31)His(B32)Gly(A21).

6. ADN codant pour un précurseur selon l'une quelconque des revendications 1 à 5.

7. Vecteur contenant un ADN selon la revendication 6.

8. Vecteur selon la revendication 7, **caractérisé en ce que** le vecteur est un vecteur d'expression convenant à l'expression chez l'espèce E. coli.

9. Cellule d'espèce E. coli contenant un vecteur selon la revendication 8.

10. Procédé pour la fabrication d'un précurseur selon l'une quelconque des revendications 1 à 5, dans lequel:
a) on incorpore un ADN selon la revendication 6 dans un vecteur selon la revendication 7;
b) on transfecte le vecteur obtenu à l'étape (a) dans une cellule d'espèce *E. coli;*
c) on utilise la cellule d'espèce *E. coli* obtenue à l'étape (b) contenant le vecteur obtenu à l'étape (a), pour la phase d'expression; et
d) on isole le précurseur à partir du surnageant de culture.

11. Procédé pour la fabrication d'un ADN selon la revendication 6, dans lequel :
a) à partir de l'ADNc de l'insuline d'origine humaine ou simienne, on produit l'ADN selon la revendication 6 par PCR et par d'autres techniques de biologie moléculaire, et
b) on l'isole.

12. Procédé pour la fabrication de l'insuline humaine ou d'un analogue de l'insuline, dans lequel :
a) un précurseur est préparé selon le procédé de la revendication 10;
b) le précurseur obtenu à l'étape (a) est soumis à un repliement dans des conditions appropriées, de manière à ce que les ponts disulfure puissent être élaborés selon la configuration de l'insuline humaine, et que la partie RDVP-Yₙ et, éventuellement, la partie de fusion, soient éliminées par voie enzymatique; et
c) l'insuline humaine, ou l'analogue d'insuline, est purifiée.

13. Utilisation d'un précurseur selon l'une quelconque des revendications 1 à 5, pour la fabrication de l'insuline ou d'un analogue de l'insuline.

14. Utilisation selon la revendication 13, dans laquelle la fabrication de l'insuline ou d'un analogue de l'insuline est effectuée selon le procédé de la revendication 12.

15. Utilisation d'un ADN selon la revendication 6 pour la fabrication d'un précurseur selon l'une quelconque des revendications 1 à 5.

16. Utilisation d'un vecteur selon la revendication 8 pour la fabrication d'un précurseur selon l'une quelconque des revendications 1 à 5.

17. Utilisation d'une cellule d'espèce E. coli selon la revendication 9 pour la fabrication d'un précurseur selon l'une quelconque des revendications 1 à 5.

18. Utilisation selon l'une quelconque des revendications 15, 16 ou 17, dans laquelle la fabrication du précurseur est effectuée selon le procédé de la revendication 10.
